# DEMANDE DE BREVET EUROPEEN

(11) **EP 0 549 394 A1**
(43) Date de publication de la demande: **30.06.1993**
(21) Numéro de dépôt: 92403349.1
(22) Date de dépôt: 09.12.1992
(51) Int. Cl.: A61M 1/16

(54) **Procédé et dispositif pour mesurer la concentration d'une substance dans un milieu complexe liquide, par exemple le sang**

(30) Priorité: 12.12.1991 FR 9115677
(71) Demandeur: HEMODIA Société Anonyme, F-31450 Fourquevaux (FR); INNOSUD, Sarl, F-31700 Blagnac (FR)
(72) Inventeur: Lacaze, Jean-Claude, F-31700 Blagnac (FR); Tremollières, Claude, F-31320 Auzeville (FR); Montoriol, Pierre, F-31520 Ramonville-Saint-Agne (FR); Aimar, Pierre, F-31400 Toulouse (FR); Montiel, Fabienne, F-31170 Tournefeuille (FR)
(74) Mandataire: Morelle, Guy Georges Alain

(57) **Abrégé**

L'invention a pour objet un procédé et un dispositif pour mesurer la concentration d'une substance dans un milieu complexe liquide circulant dans un corps, notamment le sang circulant dans le corps humain.

Ce procédé qui consiste à mettre en présence une fraction au moins dudit milieu complexe liquide avec un fluide de référence de manière à obtenir un transfert par diffusion d'une quantité de ladite susbtance vers ledit fluide de référence et à analyser ledit fluide de référence pour déterminer la concentration de ladite substance dans ledit fluide, se caractérisé par le fait ledit milieu complexe liquide est amené à circuler dans un conduit d'écoulement extra-corporel où ladite fraction dudit milieu complexe liquide est mise en présence dudit fluide de référence.

Le dispositif comprend un conduit extra-corporel 1 d'écoulement du sang, un récipient 3 pour contenir une fraction au moins de sang, monté sur un conduit de dérivation 2 bouclé sur ledit conduit d'écoulement 1, éventuellement un conduit d'amenée 4a d'un fluide de référence vers ledit récipient 3, un moyen de transfert 5 par diffusion de ladite substance vers ledit fluide de référence, ledit moyen de transfert étant contenu dans ledit récipient 3, un moyen 6 pour analyser ledit fluide de référence afin de déterminer la concentration de ladite substance, et éventuellement un conduit d'évacuation 4b dudit fluide de référence concentré en substance.

## Description

La présente invention concerne, de manière non exclusive, le matériel médico-chirurgical et intéresse plus particulièrement les systèmes de contrôle et d'analyse employés en médecine humaine notamment.

Plus précisément, l'invention a pour objet un procédé et un dispositif pour mesurer la concentration d'une substrance dans un milieu complexe liquide, par exemple le sang, et notamment lors de la mise en oeuvre d'une méthode thérapeutique telle qu'une hémodialyse.

Il existe dans l'état actuel de la technique des méthodes permettant de déterminer la concentration d'une substance présente dans un liquide complexe, mais ces dernières nécessitent obligatoirement qu'une certaine quantité dudit liquide soit prélevée sans qu'il soit possible de réintroduire ce prélèvement dans son milieu d'origine. Ceci est notamment le cas des analyses de sang qui utilisent par exemple la technique de la centrifugation.

Or, pour certaines personnes, présentant notamment des insuffisances rénales, de trop nombreuses prises de sang peuvent s'avérer préjudiciables dans la mesure où elles peuvent accentuer des déficiences. Pourtant, la conduite d'une opération d'hémodialyse, par exemple, nécessite de connaître de manière quasie permanente la composition du sang du sujet traité pour ajuster le dialysat.

Le procédé et le dispositif de l'invention permettent de déterminer le taux d'une substance dans un milieu complexe liquide, comme le sang, sans qu'aucun prélèvement soit nécessaire.

En effet, un des avantages de l'invention est de pouvoir procéder à des dosages sans perturber le milieu complexe liquide et permet d'écarter l'inconvénient que peuvent représenter de multiples prélèvements de sang pour un patient subissant régulièrement des hémodialyses.

De plus, l'invention permet de procéder à une analyse sans perturber la circulation extra-corporelle du sang, cette circulation s'effectuant en continu.

Ceci correspond à un avantage important de l'invention car il répond au besoin, jusqu'alors insatisfait, d'analyser le sang non seulement avant une séance d'hémodialyse mais aussi au cours de celle-ci afin, par exemple, pour pouvoir ajuster la composition du liquide dialyseur en fonction d'un patient donné et non plus en fonction de données générales déterminées statistiquement.

Le principe sur lequel repose le procédé de l'invention pour mesurer la concentration d'une substance dans un milieu complexe liquide circulant dans un corps, notamment le corps humain, consiste à mettre en présence une fraction au moins dudit milieu complexe liquide avec un fluide de référence de manière à obtenir un transfert par diffusion d'une quantité de ladite susbtance vers ledit fluide de référence, et à analyser ledit fluide de référence pour déterminer la concentration de ladite substance dans ledit fluide de référence, ce procédé étant caractérisé en ce qu'il consiste à faire circuler ledit milieu complexe liquide dans un conduit d'écoulement extra-corporel où ladite fraction dudit milieu complexe liquide est mise en présence dudit fluide de référence.

Ce procédé, qui est fondé ainsi sur le principe connu du transfert par diffusion, met en contact un fluide de référence, plus précisément un solvant comme de l'eau distillée, avec le liquide à analyser, le plus souvent du sang, pour rechercher notamment la concentration en Sodium dans celui-ci, l'intérêt de connaître une telle concentration étant explicité plus loin dans la description.

Le dispositif conforme à l'invention, permettant de mettre en oeuvre le procédé exposé ci-dessus, dans son application à la mesure de la concentration d'une substance donnée dans le sang, comprend :
* un conduit extra-corporel dans lequel circule en continu le sang, et relié à ses deux extrémités au corps du sujet par des moyens appropriés habituels,
* un récipient pour contenir une fraction au moins de sang, ledit récipient étant monté sur ledit conduit extra-corporel d'écoulement du sang et contenant une qualtité déterminée dudit fluide de référence ou solvant,
* un moyen de transfert par diffusion de ladite substance vers ledit fluide de référence ou solvant, ledit moyen de transfert étant contenu dans ledit récipient, et
* un moyen pour analyser ledit fluide de référence ou solvant afin de déterminer la concentration de ladite substance.

Dans une forme préférée de réalisation de ce dispositif, le susdit récipient est monté sur un conduit de dérivation bouclé sur ledit conduit extra-corporel d'écoulement pour détourner une fraction au moins du sang circulant dans ledit conduit d'écoulement, ceci sans arrêter la circulation dans ledit conduit de dérivation.

L'invention sera mieux comprise et d'autres caractéristiques et d'autres avantages la concernant apparaîtront dans la description qui suit d'exemples de réalisation d'un dispositif conforme à la définition ci-dessus donnée, ces exemples étant fournis à titre uniquement d'illustration et sans qu'aucune interprétation restrictive de la protection cherchée puisse en être tirée.

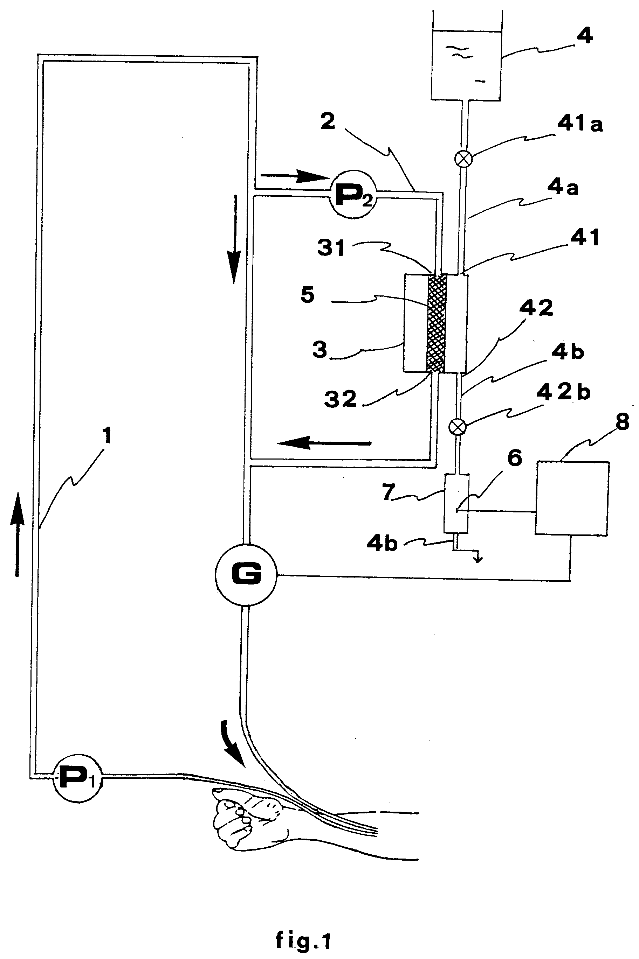

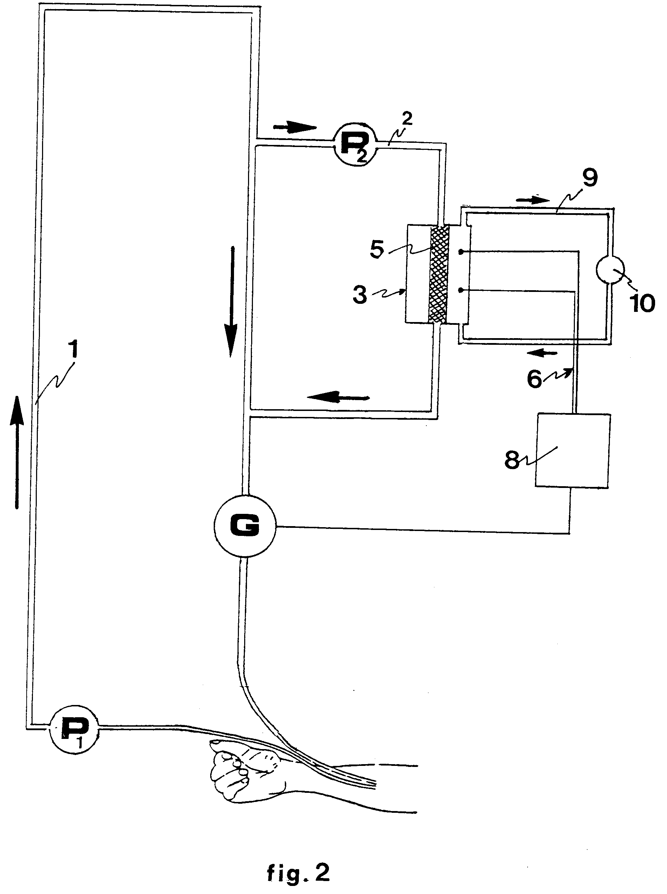

Cette description est accompagnée de dessins, sur lesquels, les Figures 1 et 2 montrent de manière schématique deux modes possibles de réalisation d'un dispositif utilisé dans le cadre d'une opération d'hémodialyse.

Dans la suite de la description, les mêmes références numériques désigneront les mêmes éléments quelle que soit la Figure où ceux-ci apparaissent.

On dispose ainsi d'un conduit 1, muni d'une pompe P1, qui assure une circulation extra-corporelle du sang dans le prolongement d'une veine d'un patient, ledit conduit amenant le sang vers un générateur de dialyse G avant de le restituer au patient.

Comme indiqué précédemment, sur ce conduit 1 est bouclé un conduit de dérivation 2. Ce conduit 2 permet ainsi de détourner à partir du conduit d'écoulement 1 une fraction de sang et amène cette fraction vers l'entrée 31 d'un récipient 3. Il se prolonge à partir de la sortie 32 dudit récipient 3, pour rejoindre le conduit d'écoulement 1. En amont du récipient, sur le conduit de dérivation 2, est disposée une pompe de circulation P2, afin de maintenir un débit suffisant dans ledit récipient 3.

Dans le mode de réalisation illustré par la Figure 1, le récipient 3 est muni également d'une seconde entrée 41 et d'une seconde sortie 42 auxquelles sont connectées respectivement une conduite 4a d'alimentation en solvant contenu dans un réservoir 4 et une conduite d'évacuation 4b dudit solvant. Ces conduites 4a, 4b sont munies de moyens d'obturation temporaire 41 a, 42b.

La mise en présence de la fraction de sang détournée par le conduit de dérivation 2 et du volume de solvant introduit dans le récipient 3 par la conduite 4a, s'effectue par l'intermédiaire d'une membrane semi-perméable 5, par exemple en polysulfone, disposée dans ledit récipient, celui-ci comportant ainsi deux compartiments. A travers la susdite membrane 5 s'opére ainsi le transfert passif des solutés présents dans le sang et notamment du Sodium, vers le volume de solvant, ce transfert étant, comme évoqué plus haut, basé sur le principe de la diffusion. En effet, la particularité du dispositif selon l'invention est d'utiliser uniquement le phénomène de diffusion.

Pour cela, un des compartiments du récipient est alimenté en une quantité de solvant prédéterminée par le volume de ce compartiment, ce qui assure un équilibre hydrostatique instantané.

Dans le mode de réalisation illustré par la Figure 2, le récipient 3 ne comporte pas de conduites d'amenée et d'évacuation du solvant. Une quantité déterminée de celui-ci a été préalablement introduite dans le compartiment qui lui est destiné, ce compartiment étant muni de moyens pour agiter le solvant. Ces moyens peuvent être des moyens d'agitation mécanique ou ultra-sonique ou plus simplement, comme suggéré par les dessins, par une conduite de recirculation 9 sur laquelle est montée une pompe 10.

La concentration en soluté du solvant que l'on désire mesurer a été initialement déterminée. Celle-ci est nulle (ou éventuellement inférieure à la concentration dudit soluté dans le sang) sachant que la quantité de soluté traversant la membrane 5 est maximale si, pour une surface efficace et un coefficient global de perméabilité de ladite membrane donnés, le solvant utilisé est dépourvu du soluté considéré.

Durant toute l'opération, le sang circule dans le conduit de dérivation 2 de manière à maintenir en permanence du sang frais au voisinage de la membrane semi-perméable 5, cette dernière constituant une barrière infranchissable dans le sens solvant-sang pour les éléments étrangers contenus dans ledit solvant, par exemple des bactéries, et dans le sens sang-solvant pour les globules rouges. Il s'opère donc uniquement un transfert par diffusion de soluté vers le solvant et une augmentation de la concentration de ce dernier en soluté jusqu'à ce que cette concentration soit égale à celle du sang, sachant que le temps nécessaire pour atteindre l'égalité des concentrations dépend du volume du compartiment où est contenu le solvant et de la surface de la membrane.

Il s'agit, dans l'exemple illustré sur les Figures, de déterminer le taux de Sodium dans le sang. En effet, cette donnée est caractéristique de la concentration des autres éléments présents dans le sang tels que le calcium, le potassium, et, par conséquent, permet de modifier, si nécessaire, la composition du bain de dialyse. Ainsi l'opération d'hémodialyse sera adaptée aux propres besoins du patient et ceci pour une meilleure purification du sang.

Il n'est pas nécessaire pour procéder au dosage d'atteindre l'égalité des concentrations en Sodium dans le solvant et dans le sang, car le relevé d'au moins trois données échelonnées dans le temps permet de déduire la courbe de saturation dudit solvant en Sodium.

Ce relevé est opéré au moyen d'un capteur de mesure 6 constitué par exemple d'au moins deux électrodes et d'un moyen de mesure de la température. Ledit capteur 6 peut être soit plongé dans le volume de solvant contenu dans le récipient 3, comme montré par la Figure 2, soit situé sur le conduit d'évacuation 4b dudit solvant concentré en substance, plus précisément au sein d'une éprouvette de mesure 7, comme montré par la Figure 1.

Un système de traitement informatique 8 est relié au susdit capteur 6, ce système agissant sur le générateur de dialyse G pour corriger, en fonction des mesures, la teneur des différents constituants du liquide dialyseur tels que sodium, potassium, magnésium, acétate etc...

Lorsque la mesure a été effectuée avec le dispositif de la Figure 1, le solvant concentré en substance est évacué par la conduite 4b soit directement du récipient 3, soit également de l'éprouvette de mesure 7, puis on initialise le compartiment qui lui est destiné en le rinçant avec le solvant provenant du réservoir 4.

A cet effet, les moyens d'obturation temporaire 41a, 42b, par exemple des électrovannes à pincement, qui commandent l'alimentation en solvant et son évacuation, sont ouvertes et laissent couler le liquide concentré par gravité jusqu'à ce que la mesure électrique indique une concentration nulle en Sodium (ou éventuellement inférieure à celle du sang). Les deux électrovannes 41a, 41b sont ensuite fermées de manière à procéder à une nouvelle mesure.

### LISTE DES REFERENCES

1. Conduit d'écoulement extra-corporel
2. Conduit de dérivation
3. Récipient
4. Réservoir de solvant
4a. Conduit d'amenée du solvant
4b. Conduit d'évacuation du solvant concentré
5. Membrane semi-perméable
6. Capteur de mesure
7. Eprouvette de mesure
8. Système de traitement informatique
9. Conduite de recirculation du solvant
10. Pompe
31. Entrée de 3 pour le conduit 2
32. Sortie de 3 pour le conduit 2
41. Entrée de 3 pour le conduit 4a
42. Sortie de 3 pour le conduit 4b
41a, 42b. Electrovannes
G. Générateur de dialyse
P1, P2. Pompes

## Revendications

1. Procédé pour mesurer la concentration d'une substance dans un milieu complexe liquide circulant dans un corps, notamment le corps humain, consistant :
* à mettre en présence une fraction au moins dudit milieu complexe liquide avec un fluide de référence de manière à obtenir un transfert par diffusion d'une quantité de ladite susbtance vers ledit fluide de référence, et
* à analyser ledit fluide de référence pour déterminer la concentration de ladite substance dans ledit fluide de référence, ***caractérisé en ce qu'***il consiste à faire circuler ledit milieu complexe liquide dans un conduit d'écoulement extra-corporel où ladite fraction dudit milieu complexe liquide est mise en présence dudit fluide de référence.

2. Procédé selon la Revendication 1, ***caractérisé en ce qu'***il consiste à détourner la susdite fraction dudit milieu complexe liquide sur un conduit de dérivation bouclé sur le susdit conduit d'écoulement extra-corporel.

3. Dispositif pour mesurer la concentration d'une substance donnée dans le sang selon le procédé des Revendications précédentes, ***caractérisé en ce qu'***il comprend :
* un conduit extra-corporel (1) d'écoulement du sang,
* un récipient (3) pour contenir une fraction au moins de sang, monté sur ledit conduit extra-corporel (1) d'écoulement du sang, ledit récipient (3) étant rempli d'une quantité déterminée d'un fluide de référence,
* un moyen de transfert (5) par diffusion de ladite substance vers ledit fluide de référence, ledit moyen de transfert étant contenu dans ledit récipient (3), et
* un moyen (6) pour analyser ledit fluide de référence afin de déterminer la concentration de ladite substance.

4. Dispositif selon la Revendication 3, ***caractérisé en ce que*** le susdit récipient (3) est monté sur un conduit de dérivation (2) bouclé sur ledit conduit d'écoulement extra-corporel (1) pour détourner une fraction au moins du sang circulant dans ledit conduit d'écoulement extra-corporel.

5. Dispositif selon la Revendication 3 ou 4, ***caractérisé en ce qu'***il comporte des moyens (9, 10) pour agiter ledit fluide référence dans ledit récipient (3).

6. Dispositif selon la Revendication 3 ou 4, ***caractérisé en ce qu'***il comporte :
* un conduit d'amenée (4a) du fluide de référence vers ledit récipient (3), et
* un conduit d'évacuation (4b) dudit fluide de référence concentré en substance.

7. Dispositif selon la Revendication 3 ou 4, ***caractérisé en ce que*** le susdit moyen de transfert par diffusion de ladite substance vers ledit fluide de référence est constitué par au moins une membrane semi-perméable (5) séparant la susdite fraction de sang et ledit fluide de référence.

8. Dispositif selon la Revendication 3, ***caractérisé en ce que*** le susdit moyen pour analyser ledit fluide de référence afin de déterminer la concentration de ladite substance comprend au moins un capteur de mesure (6).

9. Dispositif selon l'une quelconque des Revendications 3, 4 ou 8, ***caractérisé en ce que*** le susdit moyen pour analyser est disposé dans ledit récipient (3).

10. Dispositif selon la Revendication 6 ou 8, ***caractérisé en ce que*** le susdit moyen pour analyser est situé sur le susdit conduit d'évacuation (4b).

11. Dispositif selon la Revendication 8, ***caractérisé en ce que*** ledit capteur de mesure (6) est constitué par au moins deux électrodes et un moyen pour mesurer la température.

12. Dispositif selon la Revendication 5, ***caractérisé en ce que***lesdits moyens pour agiter ledit fluide de référence dans ledit récipient (3) comprennent des moyens de recirculation (9, 10).

13. Dispositif selon la Revendication 6, ***caractérisé en ce que*** ledit conduit d'amenée (4a) et ledit conduit d'évacuation (4b) du fluide de référence sont munis respectivement de moyens d'obturation temporaire (41a, 42b).

14. Dispositif selon l'une quelconque des Revendications 3 à 13, ***caractérisé en ce que*** ledit fluide de référence est un solvant.

15. Dispositif selon la Revendication 14, ***caractérisé en ce que*** ledit solvant est de l'eau distillée.

16. Dispositif selon l'une quelconque des revendications 4 à 15, ***caractérisé en ce qu'***un générateur de dialyse (G) est monté sur ledit conduit extra-corporel (1) d'écoulement du sang en aval dudit conduit de dérivation (2).
